# EUROPEAN PATENT SPECIFICATION

(11) **EP 0 225 195 B1**
(45) Date of publication and mention of the grant of the patent: **24.11.1993**
(21) Application number: 86309393.6
(22) Date of filing: 02.12.1986
(51) Int. Cl.: C07D 239/26, C07D 239/28, C09K 19/34

(54) **Phenylpyrimidine-based compounds and liquid crystal composition containing the same**
Verbindungen auf der Basis der Phenylpyrimidine und sie enthaltende flüssigkristalline Zusammensetzungen
Composés à base de phénylpyrimidines et compositions de cristaux liquides les contenant

(30) Priority: 04.12.1985 JP 272834/85
(43) Date of publication of application: 10.06.1987
(73) Proprietor: AJINOMOTO CO., INC., Tokyo 104 (JP)
(72) Inventor: Kobayashi, Toru c/o Central Res. Lab.,, Kawasaki-ku, Kawasaki-shi, Kanagawa-ken (JP); Kawashima, Hideaki c/o Central Res. Lab.,, Kawasaki-ku, Kawasaki-shi, Kanagawa-ken (JP); Tabohashi, Tatsuru c/o Central Res. Lab.,, Kawasaki-ku, Kawasaki-shi, Kanagawa-ken (JP); Sakurai, Takao c/o Central Res. Lab.,, Kawasaki-ku, Kawasaki-shi, Kanagawa-ken (JP); Higuchi, Ryoichi c/o Central Res. Lab.,, Kawasaki-ku, Kawasaki-shi, Kanagawa-ken (JP); Komatsu, Eri c/o Central Res. Lab.,, Kawasaki-ku, Kawasaki-shi, Kanagawa-ken (JP); Takeuchi, Koji c/o Central Res. Lab.,, Kawasaki-ku, Kawasaki-shi, Kanagawa-ken (JP)
(74) Representative: Bond, Bentley George

(56) References cited:
- EP-A- 0 159 872
- EP-A- 0 160 790
- EP-A- 0 186 045
- EP-A- 0 193 191
- WO-A-86/00087
- WO-A-86/06373

## Description

This invention relates to new phenylpyrimidine compounds and to liquid crystal compositions containing the same, particularly smectic liquid crystal compositions.

The phenylpyrimidine compounds herein include those compounds which are useful as a component of liquid crystal compositions even though they do not exist in the form of liquid crystals.

Twisted nematic (TN) and dynamic scatter (DS) types, both employing nematic liquid crystal cells, are the modes of liquid-crystal display most extensively used at present. One disadvantage of conventional nematic liquid crystal cells is the low response speeds (on the order of milliseconds at the highest), which limit the use of this type of liquid crystal cells.

It has recently been found that higher response speeds can be achieved by smectic liquid crystal cells and that some optically active smectic compounds are ferroelectric. Ferroelectric liquid crystals are a group of compounds which are ferroelectric when they exist as a chiral smectic C (hereinafter abbreviated as "SmC*") phase and are typified by 2-methylbutyl 4-(4-n-decyloxybenzylideneamino) cinnamate (hereinafter abbreviated as "DOBAMBC") of the following structural formula [J. Physique, 39, L-69 (1975)], 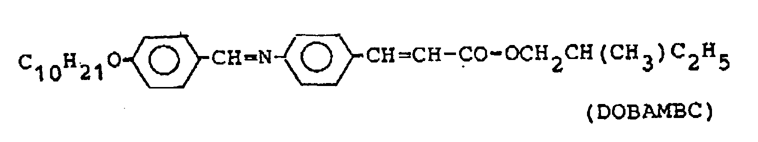
More recently N. A. Clark et al. [Appl. Phys. Lett., 36, 89 (1980)] found that very high response speeds on the order of microseconds can be achieved by thin-film DOBAMBC cells. Since then ferroelectric liquid crystals have been receiving attention not only as a display material for use in liquid-crystal TV sets, but also as an element for opto-electronic devices (e.g., photo printer heads, optical Fourier transformation elements, and light valves).

DOBAMBC remains ferroelectric only within a relatively narrow temperature range, and is unsatisfactory in physical and chemical stability because it is a Schiff base. Hence there has been a great demand for new ferroelectric compounds which are stable both physically and chemically, remain ferroelectric over a wide temperature range, have a large dielectric constant, and can be activated at a low voltage.

Furthermore, the ferroelectric compounds should have the abilities of high response, memory, alignment, high contrast and threshold voltage under practical conditions.

Pyrimidine and phenylene compounds for use in liquid crystal compositions are disclosed in EP-A-0160790 (published on the 13th November 1985), EP-A-0193191 (published on the 3rd September 1986), EP-A-0186045 (published on the 2nd July 1986), EP-A-0159872 (published on the 30th October 1985) and WO-86/06373 (published on the 6th November 1986).

However, there exist no optically active ferroelectric compounds having a larger spontaneous polarization value, a low viscosity, and a long chiral pitch and tilt angle. To solve these problems, it is possible to mix a nematic liquid crystal compound with a chiral smectic C liquid crystal compound. A compound which is able to be used as a component of a ferroelectric liquid crystal composition must have either a large spontaneous polarization value or a low viscosity, thereby either giving the composition a large spontaneous polarization value or lengthening the chiral pitch interval of an another compound in spite of having a short chiral pitch interval itself.

In one aspect of the present invention, there is provided phenylpyrimidine compound having the general formula:

R¹-A-X-B-Y-C-Z-D-R² (I)

wherein R¹ is a straight or branched C₁₋₁₈ alkyl group, a haloalkyl group, an aralkyl group or a haloaralkyl group (each with or without a chiral carbon atom); R² is a branched alkyl group containing a halogen atom on a chiral carbon atom; A is a single bond,
-O-, -COO-, -OCOO- or -OCO-; B and C are the same or different and each is a single bond,
-COO-, -OCO-, -N=CH-, -CH=N-, -CH₂O-, -OCH₂- or -CH=CH-; D is a single bond,
-COOCH₂-, -OCOO- or -OCO-; X and Z are the same or different and each is (i) 1,4-phenylene or (ii) 1,4-phenylene substituted by one or more than one halogen atom, cyano group or nitro group; and Y is 2,5-pyrimidine

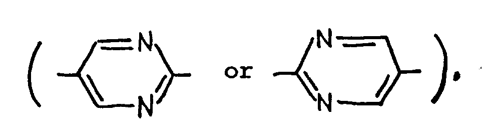

Another aspect of the present invention is a liquid crystal composition comprising at least one compound represented by Formula (I) and at least one compound selected from the following compounds (1) to (4):
(1) a smectic C liquid crystal compound without a chiral carbon atom;
(2) a smectic C liquid crystal compound without a chiral carbon atom and having the general formula (II):

   R-Ph-COO-Ph-R' (II)

   wherein R and R' are the same or different and each is an alkyl group, an alkoxy group, an acyloxy group or an alkoxycarbonyl group, and Ph is a 1,4-phenylene group;
(3) a smectic C liquid crystal compound without a chiral carbon atom and having the general formula (III)

   R-Ph-Ph-R' (III)

   wherein R and R' are the same or different and each is an alkyl group, an alkoxy group, an acyloxy group or an alkoxycarbonyl group, and Ph is a 1,4-phenylene group;
(4) a smectic C liquid compound without a chiral carbon atom and having the general formula (IV)

   R-Ph-Py-R' (IV)

   wherein R and R' are the same or different and each is an alkyl group, an alkoxy group, an acyloxy group or an alkoxycarbonyl group, Ph is a 1,4-phenylene group, and Py is a 2,5-pyrimidine group.

It is a primary object of the present invention to provide chiral smectic liquid crystal compounds which comprise both a pyrimidine group and an optically active chlorine-containing chiral group. It is believed that the former group gives the compounds a low viscosity and that the latter group gives the compound a large spontaneous polarization value.

The phenylpyrimidine compounds of the present invention are new chiral compound in themselves or are compounds which act as chiral smectic liquid crystals when mixed with other liquid crystal compounds in a desirable temperature range, and have a large spontaneous polarization value. Thus, if said compounds of the Formula I do not themselves exist in a form of a chiral smectic phase, they are nevertheless useful as a dopant for ferroelectric liquid crystals because they contain an optically active group. In this case, the compounds of Formula (I), when used as a component of a liquid crystal composition, give the composition a large spontaneous polarization value or a low viscosity. Furthermore, the compounds have the ability of lengthening the chiral pitch interval of other compounds when they are mixed with them.

Other suitable compounds for use with said compounds represented by Formula (I) are the following compounds (1) to (3):
(1) the known smectic liquid crystals which are described in Japanese Patent Applications Nos. 58-176823, 58-175711, 59-23510, 59-23511, 59-74748 (U.S. Patent No. 4592858), 59-189232 and 60-22920 (U.S. Patent Application No. 774484), 60-87034 and 60-117053 (U.S. Patent Application No. 827449), 60-144136 (U.S. Patent Application No. 878798), 60-162656, 60-162654, 60-291179, and 60-250335;
(2) smectic liquid crystals;
(3) liquid crystal compounds which have a smectic C phase when the alkyl, alkoxy, alkylcarbonyloxy or alkyloxycarbonyl groups of the above compounds (2) are reacted with a chiral carbon.

A compound of Formula (I) can be obtained by reacting a pyrimidinobenzoic chloride derivative represented by formula (V) with a phenol derivative represented by formula (VI) in a basic solvent such as pyridine:

R¹-A-X-B-Y-COCl (V)

HO-Z-D-R² (VI)

A compound of Formula (I) can also be obtained by reacting a pyrimidinophenol derivative represented by formula (VII) with a benzoic chloride derivative represented by formula (VIII) in an basic solvent such as pyridine:

R¹-A-X-B-Y-OH (VII)

ClCO-Z-D-R² (VIII)

In Formulae (V) to (VIII), R¹, R², A, B, D, X, Y and Z are as defined above in respect of Formula (I).

The optically active -DR² or R¹-A- group in formula (I) can be obtained easy from the compound such as optically active alcohols and optically active carboxylic acids; for example, 2-methylbutanol, 3-methylpentanol, 4-methylhexanol, 2-butanol, 2-pentanol, 2-hexanol, 2-heptanol, 3-hexanol, 2-octanol, 1-phenylethanol, p-(2-methylbutyl)phenol, p-(2-methylbutoxy)phenol, linalool, nerolidol, sobrerol, carbomethol, menthol, isomenthol, borneol, isoborneol, carbenol, cholesterol, 2-halo-2-phenylethanols, 2-phenyl-3-halo-1-propanol, 3-phenyl-2-halo-1-propanol, 1-phenyl-2-halo-1-propanol, 3-halo-2-methyl-1-propanol, 1,1,1-trihalo-2-propanol, 2-halo-1-butanol, 3-halo-1-butanol, 2,3-dihalo-1-butanol, 2,4-dihalo-1-butanol, 3,4-dihalo-1-butanol, 1,1,1-trihalo-2-butanol, 4,4,4-trihalo-3-halo-1-butanol, 2,3,4-trihalo-1-butanol, 3,3,4,4,4-pentahalo-2-butanol, 2-halo-3-methyl-1-butanol, 2-halo-3,3-dimethyl-1-butanol, 2-halo-1-pentanol, 3-halo-1-pentanol, 4-halo-1-pentanol, 2,4-dihalo-1-pentanol, 2,5-dihalo-1-pentanol, 1,1,1-trihalo-2-pentanol, 1,1,1,2,2-pentahalo-3-pentanol, 2-halo-3-methyl-1-pentanol, 2-halo-4-methyl-1-pentanol, 2-halo-3-monohalomethyl-4-methyl-1-pentanol, 2-halo-1-hexanol, 3-halo-1-hexanol, 4-halo-1-hexanol, 5-halo-1-hexanol, 2,5-dihalo-1-hexanol, 2,6-dihalo-1-hexanol, 1,1,1-trihalo-2-hexanol, 2,5,6-trihalo-1-hexanol, 2-halo-1-heptanol, 1,1,1-trihalo-2-heptanol, 2-halo-1-octanol and 1,1,1-trihalo-2-octanol, 2-halo-2-phenylethanoic acid, 3-halo-2-methylpropanoic acid, 2-phenyl-3-halopropanoic acid, 3-phenyl-2-methyl propanoic acid, 2-phenyl-3-halopropanoic acid, 2-halobutanoic acid, 3-halobutanoic acid, 2,3-dihalobutanoic acid, 2,4-dihalobutanoic acid, 3,4-dihalobutanoic acid, 4,4,4-trihalo-3-halobutanoic acid, 2,3,4-trihalobutanoic acid, 2-halo-3-methylbutanoic acie, 2-halo-3,3-dimethylbutanoic acid, 2-halopentanoic acid, 3-halopentanoic acid, 4-halopentanoic acid, 2,4-dihalopentanoic acid, 2,5-dihalopentanoic acid, 2-halo-3-methylpentanoic acid, 2-halo-4-methylpentanoic acid, 2-halo-3-monohalomethyl-4-methylpentanoic acid, 2-halohexanoic acid, 3-halohexanoic acid, 4-halohexanoic acid, 5-halohexanoic acid, 2,5-dihalohexanoic acid, 2,6-dihalohexanoic acid, 2,5,6-trihalohexanoic acid, 2-haloheptanoic acid, 2-halooctanoic acid, 2-phenylpropanoic acid, 2-phenylbutanoic acid, 3-phenyl-2-methylpropanoic acid, 2-methylbutanoic acid, 2,3-dimethylbutanoic acid, 2,3,3-trimethylbutanoic acid, 2-methylpentanoic acid, 3-methylpentanoic acid, 2,3-dimethylpentanoic acid, 2,4-dimethylpentanoic acid, 2,3,3,4-tetramethylpentanoic acid, 2-methylhexanoic acid, 3-methylhexanoic acid, 4-methylhexanoic acid, 2,5-dimethylhexanoic acid, 2-methylheptanoic acid, 2-methyloctanoic acid, 2-trihalomethylpentanoic acid, 2-trihalomethylhexanoic acid, 2-trihalomethylheptanoic acid, or the corresponding carboxylic acids of the above mentioned compounds.

Some of the optically active alcohols just mentioned can be easily prepared by asymmetric reduction of corresponding ketones by the action of special metal catalysts, or by microbial or enzymic action. Some other optically active acids and alcohols may be derived from optically active amino acids or oxy acids which are found in nature or obtainable by optical resolution, such as valine, leucine, isoleucine, phenylalanine, threonine, homoserine, allo-isoleucine, tert-leucine, 2-aminobutyric acid, norvaline, norleucine, ornitine, lysine, hydroxylysine, phenylglycine, aspartic acid, glutamic acid, mandelic acid, tropic acid, 3-hydroxybutyric acid, malic acid, tartaric acid, and isopropylmalic acid.

Having generally described this invention, a further understanding can be obtained by reference to certain specific examples which are provided herein for purposes of illustration only and are not intended to be limiting unless otherwise specified.

The phase transition temperature values shown in the following examples may vary slightly depending on the method used for measuring the temperature or the purity of the product.

Hereinafter following words are abbreviated as in ( ); crystal (C), chiral smectic C (SC*), smectic A (SA), chiral smectic F (F*), chiral nematic (N*), isotropic phase (I), and the carbon atom marked with * shows an asymmetric carbon atom.

### EXAMPLE 1

### Preparation and properties of (2'''S, 3'''S)-4''-(2'''chloro-3'''-methylpentyloxycarbonyl)phenyl 2'-(4-octyloxyphenyl)-pyrimidine-5'-carboxylic acid ester (A)

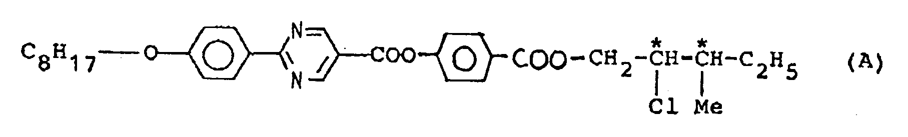

4.4g of (2'S, 3'S)-4-(2'-chloro-3'-methylpentyloxycarbonyl)phenol, sysnthesized by the method described in Japanese patent application Number 60-162656, were dissolved in 10 ml of pyridine and 200 ml of carbontetrachloride. Also, 5g of 2'-(4-octyloxyphenyl)-pyrimidine-5'-carboxylic chloride derived from p-octyloxyphenyamidine were dissolved in 300 ml of carbontetrachloride. These two solutions were mixed with each other.

After neutralizing with hydrogen chloride, the compound was extracted with chloroform solution. After removing the solution, the resulting crude compound was purified by passing through a silica gel column. 6.4 g of the compound (A) were obtained. The phase transition points of the compound were as follows, the degree in ( ) means under lowering temperature, C-SC* 119°C (107°C), SC*-SA 148°C (148°C), SA-I 167°C (167°C). The spontaneous polarization value by means of the Soya-tower method was 50 nC/cm².

The compound (A) was mixed with (2S)-2-methylbutyl 4'''-(4'-(4''-octyloxyphenylcarbonyloxy)phenyl)benzoic acid ester (B) (A:B = 42 wt%:58 wt%).

The phase transition points of the composition were as follows: C-SC*; 78°C (46°C), SC*-SA; 135°C (135°C), SA-N; 168°C (168°C), N*-I; 175°C (175°C). The spontaneous polarization value was 20 nC/cm².

The result shows that the compound of the present invention was useful as a component of composition comprising another smectic liquid crystal compound or a smectic C compound.

### EXAMPLE 2

### Preparation and properties of (1'''S, 2'''S)-4''-(1'''-chloro-2'''-methylbutylcarbonyloxy)phenyl 2'-(4-octyloxyphenyl)-pyrimidine-5'-carboxylic acid ester (C).

The compound (C) was obtained by the procedure given in example 1, using (1'S, 2'S)-4-(1'-chloro-2'-methylbutyloxycarboxy)phenol instead of (2'S, 3'S)-4-(2'-chloro-3'-methylpentyloxycarbonyl)phenol.

The phase transition points were as follows; C-SC*; 126°C (118°C), SC*-N; 148°C (148°C), N*-I; 158°C (158°C). The spontaneous polarization value was 107 nC/cm².

## Claims

1. A phenylpyrimidine compound having the general formula:
R¹-A-X-B-Y-C-Z-D-R²
wherein R¹ is a straight or branched C₁₋₁₈ alkyl group, a haloalkyl group, an aralkyl group or a haloaralkyl group (each with or without a chiral carbon atom); R² is a branched alkyl group containing a halogen atom on a chiral carbon atom; A is a single bond,
-O-, -COO-, -OCOO- or -OCO-; B and C are the same or different and each is a single bond,
-COO-, -OCO-, -N=CH-, -CH=N-, -CH₂O-, -OCH₂- or -CH=CH-; D is a single bond,
-COOCH₂-, -OCOO- or -OCO-; X and Z are the same or different and each is (i) 1,4-phenylene or (ii) 1,4-phenylene substituted by one or more than one halogen atom, cyano group or nitro group; and Y is 2,5-pyrimidine

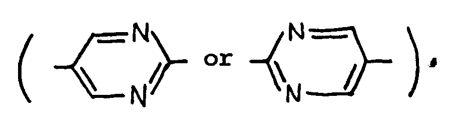

2. A liquid crystal composition containing at least one phenylpyrimidine compound as claimed in claim 1.

3. A liquid crystal composition comprising at least one phenylpyrimidine compound as claimed in claim 1 and at least one smectic C liquid crystal compound without a chiral carbon atom.

4. A liquid crystal composition as claimed in claim 3, wherein said at least one smectic C liquid crystal compound without a chiral carbon atom has the general formula:
R-Ph-COO-Ph-R'
wherein R and R' are the same or different and each is an alkyl group, an alkoxy group, an acyloxy group or an alkoxycarbonyl group, and Ph is a 1,4-phenylene group.

5. A liquid crystal composition as claimed in claim 3, wherein said at least one smectic C liquid crystal compound without a chiral carbon atom has the general formula:
R-Ph-Ph-R'
wherein R and R' are the same or different and each is an alkyl group, an alkoxy group, an acyloxy group or an alkoxycarbonyl group, and Ph is a 1,4-phenylene group.

6. A liquid crystal composition as claimed in claim 3, wherein said at least one smectic C liquid crystal compound without a chiral carbon atom has the general formula:
R-Ph-Py-R'
wherein R and R' are the same or different and each is an alkyl group, an alkoxy group, an acyloxy group or an alkoxycarbonyl group, Ph is a 1,4-phenylene group, and Py is a 2,5-pyrimidine group.

## Patentansprüche

1. Phenylpyrimidin-Verbindung der allgemeinen Formel:
R¹-A-X-B-Y-C-Z-D-R²,
worin R¹ eine geradkettige oder verzweigte C₁₋₁₈-Alkylgruppe, eine Halogenalkylgruppe, eine Aralkylgruppe oder eine Halogenaralkylgruppe (jeweils mit einem oder ohne ein chirales Kohlenstoffatom) ist;
R² eine verzweigte Alkylgruppe, welche ein Halogenatom an einem chiralen Kohlenstoffatom enthält, ist;
A eine Einfachbindung, -O-, -COO-, -OCOO- oder -OCO- ist;
B und C gleich oder verschieden sind und jeweils eine Einfachbindung, -COO-, -OCO-, -N=CH-, -CH=N-, -CH₂O-, -OCH₂- oder -CH=CH- sind;
D eine Einfachbindung, -COOCH₂-, -OCOO- oder -OCO- ist;
X und Z gleich oder verschieden sind und jeweils (i) 1,4-Phenylen oder (ii) 1,4-Phenylen, welches durch ein oder mehrere Halogenatome, Cyangruppen oder Nitrogruppen substituiert ist; und
Y
2,5-Pyrimidin, 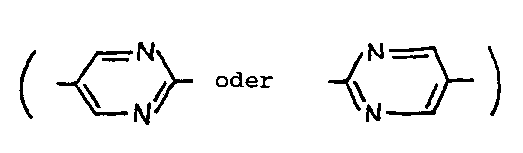 ist.

2. Flüssigkristall-Zusammensetzung, welche mindestens eine Phenylpyrimidin-Verbindung gemäß Anspruch 1 enthält.

3. Flüssigkristall-Zusammensetzung, welche mindestens eine Phenylpyrimidin-Verbindung gemäß Anspruch 1 und mindestens eine smektische C-Flüssigkristall-Verbindung ohne chirales Kohlenstoffatom enthält.

4. Flüssigkristall-Zusammensetzung gemäß Anspruch 3, worin die mindestens eine smektische C-Flüssigkristall-Verbindung ohne chirales Kohlenstoffatom die allgemeine Formel:
R-Ph-COO-Ph-R'
besitzt, worin
R und R' gleich oder verschieden sind und jeweils eine Alkylgruppe, eine Alkoxygruppe, eine Acyloxygruppe oder eine Alkoxycarbonylgruppe sind, und
Ph eine 1,4-Phenylengruppe ist.

5. Flüssigkristall-Zusammensetzung gemäß Anspruch 3, worin die mindestens eine smektische C-Flüssigkristall-Verbindung ohne chirales Kohlenstoffatom die allgemeine Formel:
R-Ph-Ph-R'
besitzt, worin
R und R' gleich oder verschieden sind und jeweils eine Alkylgruppe, eine Alkoxygruppe, eine Acyloxygruppe oder eine Alkoxycarbonylgruppe sind, und
Ph eine 1,4-Phenylengruppe ist.

6. Flüssigkristall-Zusammensetzung gemäß Anspruch 3, worin die mindestens eine smektische C-Flüssigkristall-Verbindung ohne chirales Kohlenstoffatom die allgemeine Formel:
R-Ph-Py-R'
besitzt, worin
R und R' gleich oder verschieden sind und jeweils eine Alkylgruppe, eine Alkoxygruppe, eine Acyloxygruppe oder eine Alkoxycarbonylgruppe sind, und
Ph eine 1,4-Phenylengruppe ist, und
Py eine 2,5-Pyrimidingruppe ist.

## Revendications

1. Composé de phénylpyrimidine ayant la formule générale:
R¹-A-X-B-Y-C-Z-D-R² (I)
dans laquelle R¹ est un groupe alkyle linéaire ou ramifié en C₁-C₁₈, un groupe halogénoalkyle, un groupe aralkyle ou un groupe halogénoaralkyle (chaque groupe contenant ou non un atome de carbone chiral); R² est un groupe alkyle ramifié contenant un atome d'halogène sur un atome de carbone chiral; A est une liaison simple, -O-, -COO-, -OCOO- ou -OCO-; B et C sont identiques ou différents et représentent chacun une liaison simple, -COO-, -OCO-, -N=CH-, -CH=N-, -CH₂O-, -OCH₂- ou -CH=CH-; D est une liaison simple, -COOCH₂-, -OCOO-ou -OCO-; X et Z sont identiques ou différents et représentent chacun (i) un 1,4-phénylène ou (ii) un 1,4-phénylène substitué par ou ou plusieurs atomes d'halogène, groupes cyano ou groupes nitro; et Y est une 2,5-pyrimidine

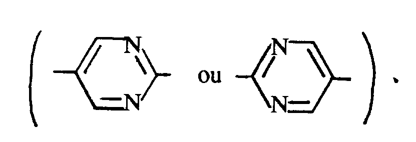

2. Composition de cristaux liquides contenant au moins un composé de phénylpyrimidine selon la revendication 1.

3. Composition de cristaux liquides comprenant au moins un composé de phénylpyrimidine selon la revendication 1 et au moins un composé de cristal liquide smectique C sans atome de carbone chiral.

4. Composition de cristaux liquides selon la revendication 3, dans laquelle ledit composé de cristal liquide smectique C sans atome de carbone chiral a la formule générale:
R-Ph-COO-Ph-R'
dans laquelle R et R' sont identiques ou différents et représentent chacun un groupe alkyle, un groupe alcoxy, un groupe acyloxy ou un groupe alcoxycarbonyle, et Ph est un groupe 1,4-phénylène.

5. Composition de cristaux liquides selon la revendication 3, dans laquelle ledit composé de cristal liquide smectique C sans atome de carbone chiral a la formule générale:
R-Ph-Ph-R'
dans laquelle R et R' sont identiques ou différents et représentent chacun un groupe alkyle, un groupe alcoxy, un groupe acyloxy ou un groupe alcoxycarbonyle, et Ph est un groupe 1,4-phénylène.

6. Composition de cristaux liquides selon la revendication 3, dans laquelle ledit composé de cristal liquide smectique C sans atome de carbone chiral a la formule générale:
R-Ph-Py-R'
dans laquelle R et R' sont identiques ou différents et représentent chacun un groupe alkyle, un groupe alcoxy, un groupe acyloxy ou un groupe alcoxycarbonyle, Ph est un groupe 1,4-phénylène, et Py est un groupe 2,5-pyrimidine.
